# EUROPEAN PATENT SPECIFICATION

(11) **EP 4 121 744 B1**
(45) Date of publication and mention of the grant of the patent: **24.06.2026**
(21) Application number: 21720328.0
(22) Date of filing: 22.03.2021
(51) Int. Cl.: G01N 21/64, G01N 21/77, G01N 33/543, G01N 33/58

(54) **OPTICAL SENSOR AND METHOD FOR DETECTING A TARGET PRESENT IN A SAMPLE FLUID**
OPTISCHER SENSOR UND VERFAHREN ZUR ERKENNUNG EINES IN EINER PROBENFLÜSSIGKEIT VORHANDENEN ANALYTEN
CAPTEUR OPTIQUE ET PROCEDE DE DETECTION D'UNE CIBLE PRESENTE DANS UN FLUIDE ECHANTILLON

(30) Priority: 20.03.2020 US 202062992511 P
(43) Date of publication of application: 25.01.2023
(73) Proprietor: LUMIRADX UK LTD, London SE1 2AQ (GB)
(72) Inventor: BRUIN, Amanda N., Solana Beach, California 92075 (US); WILLMORE, Matthew J., Solana Beach, California 92075 (US); ROSE, Tristin E., Solana Beach, California 92075 (US); MARTINEZ-ARIZA, Guillermo, Solana Beach, California 92075 (US); BODDUPALLY, Keerthi, Solana Beach, California 92075 (US); FIACCO, Stephen V., Solana Beach, California 92075 (US)
(74) Representative: Marks & Clerk LLP
(86) International application number: PCT/US2021/023530
(87) International publication number: WO 2021/189057

(56) References cited:
- WO-A1-2015/179725
- US-A1- 2012 197 231
- US-A1- 2017 030 875
- US-A1- 2019 357 825

## Description

### FIELD OF THE INVENTION

The present invention relates to optical sensors.

### BACKGROUND

Optical sensors may be used to determine the presence and/or amount of a target present in a sample liquid. Exemplary targets include biological molecules such as proteins or small molecules, and inorganic targets such as physiologically important ions. A sensor may include an optical reporter (e.g. a luminescent or fluorescent reagent) that provides a luminescent or fluorescent reagent signal, e.g., fluorescence, indicative of the presence and/or amount of the target. For example, the amount of fluorescence emitted by a luminescent or fluorescent reagent may change and/or shift in wavelength in the presence of the target. Alternatively, or in addition, the luminescent or fluorescent reagent signal may be indicative of the presence and/or amount of an indicator species that itself is indicative of the presence and/or amount of target. For example, the target may be a substrate of an enzyme and the indicator species may be an enzymatic reaction product produced by the enzyme in the presence of the luminescent or fluorescent reagent. An optical sensor may also include an optical reference that provides an optical reference signal, e.g., fluorescence, generally independent of the presence and/or amount of target or indicator species. The optical reference signal may be used to adjust or correct the luminescent or fluorescent reagent signal for, e.g., variations in the amounts of reagents or sensor response.

US 2012/0197231 A1 relates to an implantable medical device with a pulse generator and a chemical sensor in communication with the pulse generator, the chemical sensor configured to detect an ion concentration in a bodily fluid. The disclosure includes a method for providing cardiac arrhythmia therapy to a patient including sensing a physiological concentration of an analyte, communicating data regarding the physiological concentration of the analyte to an implanted pulse generator, and delivering therapy to the patient based in part on the physiological concentration of the ion. The disclosure includes a method for monitoring diuretic therapy. The disclosure includes a method for controlling delivery of an active agent into a human body.

### SUMMARY OF THE INVENTION

An optical sensor for detecting a target present in a sample fluid, comprising: a detection chamber configured to receive the sample fluid; a single polymer layer disposed in the detection chamber, the single polymer layer comprising: (i) a luminescent or fluorescent reagent that is covalently bound to a polymer of the single polymer layer and is configured to generate a luminescent or fluorescent reagent signal indicative of the presence of the target within the single polymer layer when the single polymer layer is irradiated with excitation light from an excitation source, (ii) an optical isolating agent, and (iii) an optical reference that is covalently bound to a polymer of the single polymer layer and is configured to generate an optical reagent signal generally independent of the presence of the target within the single polymer layer when the single polymer layer is irradiated with the excitation light from the excitation source, and wherein the single polymer layer (iv) is at least semi-permeable to the target, (v) has a first surface configured to contact sample fluid present in the detection chamber, (vi) has a second surface secured to a support that is optically transparent at wavelengths useful for exciting and detecting luminescence or fluorescence from the luminescent or fluorescent reagent and/or the optical reference present therein, (vii) has a transmittance of about 20% or less at a peak excitation wavelength of the luminescent or fluorescent reagent along an optical axis that is normal to the second surface of the single polymer layer.

A method of detecting a target present in a sample fluid, the method comprising: contacting a first surface of a single polymer layer with the sample fluid, the single polymer layer being at least semi-permeable to the target and comprising (i) a luminescent or fluorescent reagent covalently bound to a polymer of the single polymer layer and configured to generate a luminescent or fluorescent reagent signal indicative of the presence of the target within the single polymer layer when the single polymer layer is irradiated with excitation light from an excitation source, (ii) an optical isolating agent and (iii) an optical reference covalently bound to a polymer of the single polymer layer and configured to generate an optical reference signal generally independent of the presence of the target within the sample fluid when the single polymer layer is irradiated with excitation light from an excitation source, irradiating the single polymer layer with the excitation light along an optical axis that passes into the single polymer layer through a second surface of the single polymer layer not in contact with the sample fluid, the optical axis being normal to the second surface of the single polymer layer, wherein the second surface is secured to a support that is optically transparent at wavelengths useful for exciting and detecting luminescence or fluorescence from a luminescent or fluorescent reagent and/or an optical reference present therein, and the single polymer layer transmitting about 20% or less at a peak excitation wavelength of the luminescent or fluorescent reagent along the optical axis.

In embodiments, a luminescent or fluorescent reagent providing a luminescent or fluorescent reagent signal, e.g., fluorescence, indicative of the presence and/or amount of sodium ion (Na⁺) includes a compound having the general formula:

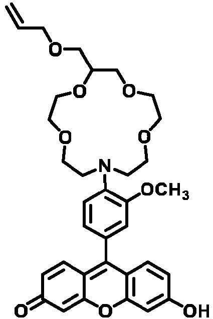

In embodiments, a luminescent or fluorescent reagent providing a luminescent or fluorescent reagent signal, e.g., fluorescence, indicative of the presence and/or amount of potassium ion (K⁺) includes a compound having the general formula:

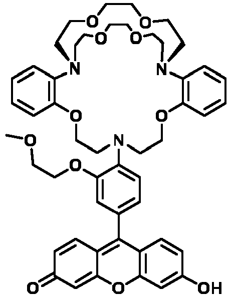

In embodiments, a luminescent or fluorescent reagent providing a luminescent or fluorescent reagent signal, e.g., fluorescence, indicative of the presence and/or amount of potassium ion (K⁺) includes a compound having the general formula:

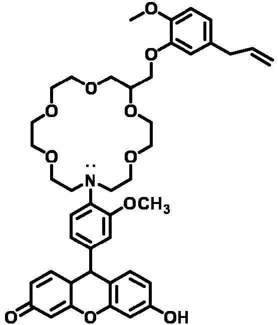

In embodiments, a luminescent or fluorescent reagent providing a luminescent or fluorescent reagent signal, e.g., fluorescence, indicative of the presence and/or amount of calcium ion (Ca⁺⁺) includes a compound having the general formula:

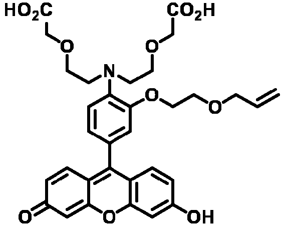

In embodiments, a luminescent or fluorescent reagent providing a luminescent or fluorescent reagent signal, e.g., fluorescence, indicative of the presence and/or amount of hydrogen ion (H⁺), e.g., a pH sensor, includes a compound having the general formula:

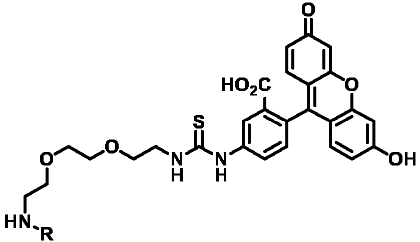

In embodiments, a luminescent or fluorescent reagent includes a derivative of any of the forgoing luminescent or fluorescent reagents. In some embodiments, the derivative includes one of the forgoing luminescent or fluorescent reagents with one or more hydrogen atoms substituted with a group selected from an aliphatic group, a lipophilic group, a hydrophilic group, an electron withdrawing group, an electron donating group, or a combination thereof. For example, a derivative of any of the forgoing luminescent or fluorescent reagents may include a luminescent or fluorescent reagent substituted with such a group or groups, e.g., a halogen, at the 2 and/or 7 position of the xanthene group of the luminescent or fluorescent reagent. An example of such a derivative is a luminescent or fluorescent reagent providing a luminescent or fluorescent reagent signal, e.g., fluorescence, indicative of the presence and/or amount of calcium ion (Ca⁺⁺) includes a compound having the general formula and a fluorine substituted for hydrogen at the 2 and/or 7 position of the xanthene group of the luminescent or fluorescent reagent:

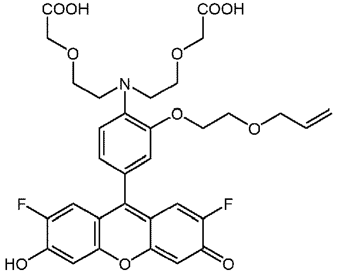

In some embodiments, the derivative excludes a reactive group for coupling to a luminescent or fluorescent reagent or to a polymer along which the luminescent or fluorescent reagent is bound. In some embodiments, the derivative includes a reactive group with a modified reactive moiety and/or a reactive group including a modified linking group connecting the reactive group to the luminescent or fluorescent reagent.

In embodiments, an optical sensor is configured for the determination of the presence and/or amount of a target in a sample liquid. The optical sensor is configured in the form of a single polymer layer, e.g., a layer of a hydrophilic polymer such as a urethane, e.g., an ether-based hydrophilic urethane. A first surface of the single polymer layer is configured to be exposed to a sample fluid, e.g., a sample liquid, containing a target of interest. A second surface of the single polymer layer is secured to a support that is optically transparent at wavelengths useful for exciting and detecting luminescence, fluorescence, from a luminescent or fluorescent reagent and or optical reference present therein. The single polymer layer is permeable with respect to the target such that, when the first surface of the single polymer layer contacts the sample liquid, target present in the liquid passes through the first surface into the single polymer layer, e.g., by diffusion, and interacts with the luminescent or fluorescent reagent therein. A composition of the single polymer layer, e.g., the composition of the polymer of the single polymer layer and/or the concentration of one or more of the luminescent or fluorescent reagent, optical reference, and optical isolating agent may be generally homogeneous proceeding between the first surface configured to be in contact with the sample liquid and the second surface secured to the optical support.

In some embodiments, the optical sensor includes at least one of the foregoing luminescent or fluorescent reagents or derivative thereof. For example, the luminescent or fluorescent reagent may be covalently bound to or physically constrained within a single polymer layer of the optical sensor. The polymer may be a hydrophilic polymer such as a urethane, e.g., an ether-based hydrophilic urethane.

In some embodiments, the target is an inorganic ion, e.g., Ca⁺⁺, K⁺, Na⁺, or H⁺. In some embodiments, the target is a small molecule, e.g., creatinine, lactate, or glucose. In some embodiments, the target is a protein.

In some examples of the disclosure, the optical sensor includes, within a single optical sensing layer, e.g., a single polymer layer, a combination of a luminescent or fluorescent reagent and an optical isolating agent, e.g., carbon black. The luminescent or fluorescent reagent and optical isolating agent may be distributed generally homogeneously within the single polymer layer. In some embodiments, the single polymer layer including the luminescent or fluorescent reagent and optical isolating agent is the only layer of the optical sensor that participates in the determination of the presence and/or amount of a target in the sample liquid. The single polymer layer may have a first surface that contacts the sample liquid and a second surface adhered to an optically transparent support.

The optical sensor may include a light source configured to irradiate the single polymer layer with excitation light to excite fluorescence from the luminescent or fluorescent reagent within the single polymer layer and a detector configured to detect the fluorescence emitted from said excited luminescent or fluorescent reagent within the single polymer layer. In some embodiments, a first surface of the single polymer layer is configured to be exposed to be in direct contact with a sample fluid, e.g., a sample liquid, and an opposed second surface of the single polymer layer is secured to an optical window through which the light source directs the excitation light and the detector detects fluorescence. In some embodiments, the single polymer layer is configured to attenuate an average of at least about 85%, at least about 90%, at least about 95%, at least about 98%, or at least about 99% of the excitation light impinging upon the second surface of the single polymer layer prior to the excitation light reaching the first surface thereof. In some embodiments, the single polymer layer is configured to attenuate an average of at least about 50%, at least about 75%, at least about 85%, at least about 90%, at least about 95%, at least about 98%, or at least about 99% of light that is emitted at the first surface within a fluorescence wavelength range detected by the detector prior to such light passing through the single polymer layer and through the second surface thereof. The attenuation of the light may be determined along an optical axis that is normal to the second surface of the single polymer layer.

In some embodiments, the single polymer layer has a thickness, in the dry state, of about 250 µm or less, about 125 µm or less, about 75 µm or less, about 50 µm or less, or about 30 µm or less. The single polymer layer may have a thickness, in the dry state, of at least about 5 µm, at least about 10 µm, at least about 20 µm, or at least about 25 µm. The thickness of the single polymer layer may be determined along an axis normal to a surface of the single polymer layer configured to be disposed in contact with a sample liquid. The thickness of the single polymer layer may change by about 50% or less, about 35% or less, about 25% or less, about 20% or less, or about 15% or less upon exposure to a sample liquid.

In some examples of the disclosure, the optical sensor includes an optical medium, e.g., a polymeric medium, that is a mixture including a luminescent or fluorescent reagent emitting light, e.g., fluorescence, within an emission wavelength band and an optical isolating agent absorbing light within the emission wavelength band. The optical medium may be configured as a layer, e.g., a single polymer layer, with a second surface secured with respect to an optically transmissive support and a first opposing surface accessible to the target or an indicator species indicative of the target present in the sample liquid. A light source is configured to direct excitation light through the optically transmissive support and irradiate the luminescent or fluorescent reagent residing within the optical medium with the excitation light. A detector is configured to detect light within a detection light band that is emitted by the luminescent or fluorescent reagent from within the layer and passes through the optically transmissive support. The layer defines a thickness between the first and second surfaces thereof along an axis normal to the second surface. The average transmission over the detection light band of the layer over the thickness is about 50% or less, about 25% or less, about 15% or less, about 10% or less, about 5% or less, about 2% or less, or about 1% or less. The average transmission detection light band of the layer over the thickness may be, e.g., at least about 0.25%, at least about 0.5%, at least about 1%, at least about 2%, or at least about 2.5%. The optical isolating agent may be carbon black. The optical isolating agent may be carbon black. In some embodiments, the first surface is in direct contact with a surface of the optically transmissive support and the second surface is configured to be placed within direct contact with a sample liquid.

In some examples of the disclosure, the optical sensor includes an optical medium that is a mixture including a luminescent or fluorescent reagent emitting light, e.g., fluorescence, when excited with light having a wavelength within an excitation wavelength band and an optical isolating agent absorbing light within the excitation wavelength band. The optical medium may be configured as a layer, e.g., a single polymer layer as in the present invention, with a second surface secured with respect to an optically transmissive support and a first opposing surface accessible the target or an indicator species indicative of the target present in the sample liquid. A light source is configured to direct excitation light within the excitation wavelength band through the optically transmissive support and irradiate the luminescent or fluorescent reagent residing within the optical medium with the excitation light. A detector is configured to detect light that is emitted by the luminescent or fluorescent reagent from within the layer and passes through the optically transmissive support. The layer defines a thickness between the first and second surfaces thereof along an axis normal to the second surface. The average transmission over the excitation light band of the layer over the thickness is about 50% or less, about 25% or less, about 15% or less, about 10% or less, about 5% or less, about 2% or less, or about 1% or less. The average transmission over the excitation light band of the layer over the thickness may be, e.g., at least about 0.25%, at least about 0.5%, at least about 1%, at least about 2%, or at least about 2.5%. The optical isolating agent may be carbon black. In some embodiments, the first surface is in direct contact with a surface of the optically transmissive support and the second surface is configured to be placed within direct contact with a sample liquid.

In some examples of the disclosure, the optical sensor includes an optical medium that is a mixture including a luminescent or fluorescent reagent emitting light, e.g., fluorescence, within an emission wavelength band when excited with light having a wavelength within an excitation wavelength band and an optical isolating agent absorbing light within the emission wavelength band and the excitation wavelength band. The optical medium may be configured as a layer, e.g., a single polymer layer, with a second surface secured with respect to an optically transmissive support and a first opposing surface accessible to the target or an indicator species indicative of the target present in the sample liquid. A light source is configured to direct excitation light within the excitation wavelength band through the optically transmissive support and irradiate the luminescent or fluorescent reagent residing within the optical medium with the excitation light. A detector is configured to detect light within a detection light band that is emitted by the luminescent or fluorescent reagent from within the layer and passes through the optically transmissive support. The layer defines a thickness between the first and second surfaces thereof along an axis normal to the second surface. The average transmission over the excitation wavelength band and the detection light band of the layer over the thickness is about 50% or less, about 25% or less, about 15% or less, about 10% or less, about 5% or less, about 2% or less, or about 1% or less. The optical isolating agent may be carbon black. The average transmission over the excitation wavelength band and the detection light band of the layer over the thickness may be, e.g., at least about 0.25%, at least about 0.5%, at least about 1%, at least about 2%, or at least about 2.5%. The optical isolating agent may be carbon black. In some embodiments, the first surface is in direct contact with a surface of the optically transmissive support and the second surface is configured to be placed within direct contact with a sample liquid.

In embodiments, the single polymer layer comprises a mixture of a luminescent or fluorescent reagent molecule and carbon black, wherein the carbon black is present in an amount of at least about 0.05%, at least about 0.1%, at least about 0.5%, at least about 1%, at least about 2%, at least about 3%, at least about 4%, or at least about 5% by weight of the single polymer layer. The carbon black may be present in an amount, e.g., of about 5% or less, about 4% or less, about 3% or less, about 3% or less, about 2% or less, or about 1% or less by weight of the single polymer layer. The optical sensor may be configured such that substantially all, essentially all, or the entirety of a luminescent or fluorescent reagent signal generated by the luminescent or fluorescent reagent molecule in the optical sensor arises from the luminescent or fluorescent reagent molecule in the single polymer layer including the carbon black. The optical sensor may exclude essentially any, e.g., any of the luminescent or fluorescent reagent that resides apart from the single polymer layer that also includes the carbon black. A first surface of the single polymer layer is configured to be placed in direct contact with a sample liquid and a second opposing surface of the single polymer layer is in direct contact with a surface of an optically transmissive support through which the single polymer layer can be irradiated with light to excite fluorescence from the luminescent or fluorescent reagent and through which fluorescence emitted by the luminescent or fluorescent reagent from within the single polymer layer can be detected.

In embodiments, an optical sensor for detecting a target present in a sample fluid comprises the features defined in appended claim 1, including a single polymer layer, the single polymer layer including a luminescent or fluorescent reagent and an optical isolating agent, such as carbon black and, an optical reference, the single polymer layer having a first surface configured to contact the sample liquid and a second surface secured to an optically transparent support, wherein the optical isolating agent, such as carbon black may be present in an amount of at least about 0.1%, 0.2%, 0.3%, 0.4%, 0.5%, 0.6%, 0.7%, 0.8%, 0.9%, or 1% by weight of the single polymer layer. In embodiments, a method of using the optical sensor includes the features defined in appended claim 8, including contacting the first surface of the single polymer layer with a sample liquid whereupon at least some of the target passes into the single polymer layer and interacts with the luminescent or fluorescent reagent. The single polymer layer is irradiated with light through the second surface to excite fluorescence from the luminescent or fluorescent reagent. The fluorescence is detected and the presence and/or amount of target present in the sample liquid is determined based on the detected fluorescence.

In embodiments, an optical sensor for detecting a target present in a sample liquid comprises the features defined in appended claim 1, including a single polymer layer, the single polymer layer including a luminescent or fluorescent reagent and an optical isolating agent, such as carbon black and, an optical reference, the single polymer layer having a first surface configured to contact the sample liquid and a second surface secured to an optically transparent support, wherein the carbon black is present in an amount sufficient to reduce the optical transmittance of light traveling between the first and second surfaces of the single polymer layer along an optical axis normal to the second surface to less than about 20%, less than about 15%, less than about 10%, less than about 5%, or less than about 2.5% of the transmittance of the layer in the absence of the carbon black at a wavelength of 475 nm. In embodiments, a method of using the optical sensor includes contacting the first surface of the single polymer layer with a sample liquid whereupon at least some of the target passes into the single polymer layer and interacts with the luminescent or fluorescent reagent. The single polymer layer is irradiated with light through the second surface to excite fluorescence from the luminescent or fluorescent reagent. The fluorescence is detected and the presence and/or amount of target present in the sample liquid is determined based on the detected fluorescence.

Any of the optical sensors disclosed herein may be disposed in a detection zone of a microfluidic strip such as a microfluidic strip disclosed in International application no. PCT/GB2017/051946 filed 30 June 2017 the ("'946 application") or International Application No. PCT/US2021/013325 with an international filing date of January 13, 2021 ("'325 application). In such embodiments, an internal surface of a wall of the detection zone of the microfluidic strip is an optical support for the optical sensor. In use, the optical sensor is irradiated with excitation light through the wall of the strip and fluorescence generated by the luminescent or fluorescent reagent and/or optical reference passes through the wall of the strip and is detected. The presence and/or amount of the target is determined based on the detected fluorescence.

In examples of the disclosure, a method of forming a polymer including a luminescent or fluorescent reagent includes combining a one or more polymerization monomers and a luminescent or fluorescent reagent having a polymerization group polymerizable with such monomers to form a polymer having the luminescent or fluorescent reagent covalently bound therealong. The luminescent or fluorescent reagent may include, for example, any of the luminescent or fluorescent reagents disclosed herein. The polymerization group of the luminescent or fluorescent reagent may include a vinyl group. The monomers may each include a vinyl group. For example, a polymer useful in the manufacture of a sensor directed to determination of the presence and/or amount of Na⁺ may be formed according to the following reaction in the presence of azobisisobutyronitrile (AIBN) and THF:

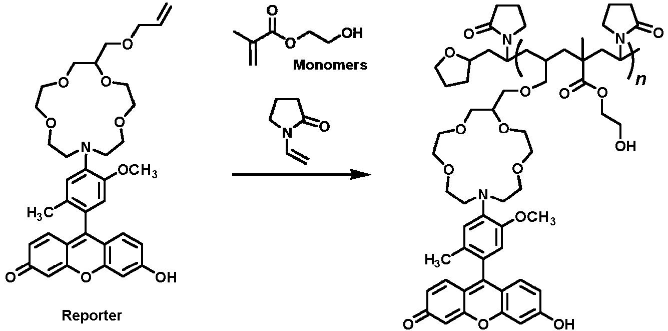

In some examples of the disclosure, the method of forming the polymer including a luminescent or fluorescent reagent further includes combining an optical reference with the monomers and luminescent or fluorescent reagent such that the formed polymer further includes the luminescent or fluorescent reagent covalently bound therealong. The optical reference is a compound having a known, e.g., constant, optical response as a function of concentration of the target to which the optical sensor is directed. For example, a polymer useful in the manufacture of a sensor directed to determination of the presence and/or amount of Na⁺ may be formed according to the following reaction in the presence of azobisisobutyronitrile (AIBN) and THF:

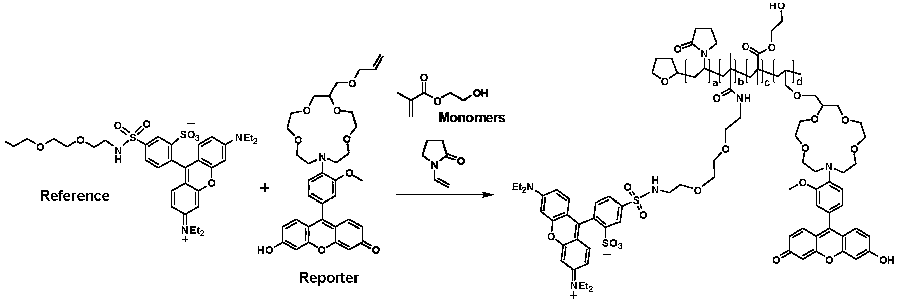

In examples of the disclosure, a method of forming a polymer including an optical reference includes combining a one or more polymerization monomers and a luminescent or fluorescent reagent having a polymerization group polymerizable with such monomers to form a polymer having the optical reference covalently bound therealong. The optical reference is a compound having a known, e.g., constant, optical response as a function of concentration of the target to which the optical sensor is directed. The polymer may exclude a luminescent or fluorescent reagent. For example, a polymer containing an optical reference may be formed according to the following reaction in the presence of azobisisobutyronitrile (AIBN) and THF:

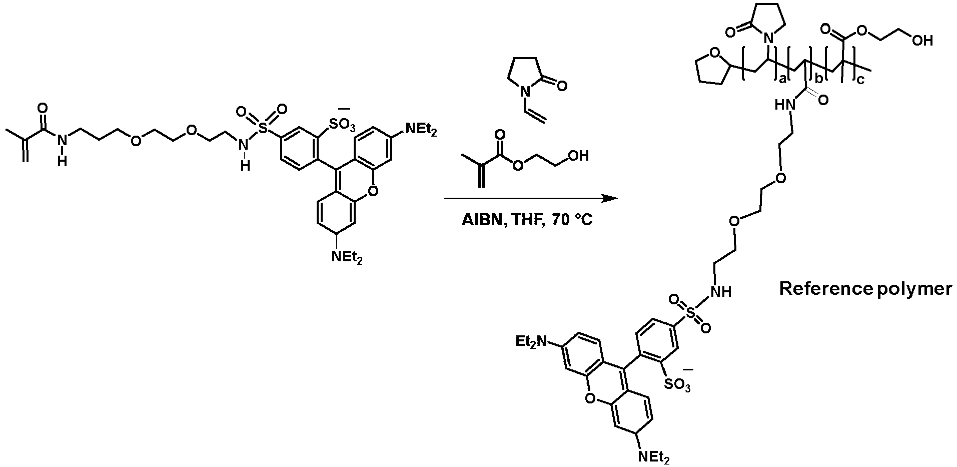

In examples of the disclosure, a method of manufacturing an optical sensor medium includes forming a mixture including (i) a hydrophilic polymer; (ii) an optical isolating agent; (iii) a luminescent or fluorescent reagent; (iv) an optical reference; and (v) the remainder to 100% solvent, with the percentages being by weight with respect to the total weight of the formed mixture. For example, the formed mixture may include (i) between about 8% to 30% by weight hydrophilic polymer; (ii) between about 0.1% to 5% optical isolating agent; (iii) between about 5 x 10⁻⁵% to 8 x 10⁻²% luminescent or fluorescent reagent; (iv) between about 0% to 8 x 10⁻²% optical reference; and (v) the remainder to 100% solvent. A ratio of the weight of luminescent or fluorescent reagent to the weight of optical reference may be, e.g., at least about 1:1, at least about 2:1, at least about 3:1, or at least about 4:1. The ratio of the weight of luminescent or fluorescent reagent to the weight of optical reference may be, e.g., about 1:1 or less, about 1:2 or less, about 1:3 or less, or about 1:4 or less.

In some examples of the disclosure, the hydrophilic polymer includes a first portion of polymer that is free of luminescent or fluorescent reagent or optical reference molecules and a second portion of polymer that includes both luminescent or fluorescent reagent and optical reference molecules covalently bound therealong. For example, the first portion of luminescent or fluorescent reagent may be between about 0% to 22% and the second portion of polymer may be between about 0.1 and 8% in each case by weight of the total mixture. In some embodiments, the hydrophilic polymer includes a first portion of polymer that is free of luminescent or fluorescent reagent or optical reference molecules, a second portion of polymer that includes a luminescent or fluorescent reagent, but no optical reference, covalently bound therealong, and a third portion of polymer that includes an optical reference, but no luminescent or fluorescent reagent, covalently bound therealong. For example, the first portion of luminescent or fluorescent reagent may be between about 0% to 22%, the second portion of polymer may be between about 0.1 and 4%, and the third portion of luminescent or fluorescent reagent may be between about 0.1 and 4%, in each case by weight of the total mixture.

In some embodiments, the luminescent or fluorescent reagent is covalently bound to at least a portion of the hydrophilic polymer. The mixture may include a first portion of hydrophilic polymer that is free of the luminescent or fluorescent reagent and a second portion of hydrophilic polymer including, e.g., covalently bound therein or physically entrapped therein, the luminescent or fluorescent reagent. The first portion of polymer may be between about 0.5% to 15% by weight of the mixture and the second portion of the polymer may be between about 0.5% and 15% by weight of the mixture. The luminescent or fluorescent reagent may be between about 0.5% to 8% by weight with respect to the total weight of the second portion of polymer.

The optical isolating agent may be, e.g., a particulate, e.g., carbon black or a carbon nanostructure such as a carbon nanotube and/or a compound such as a molecule having a high optical absorbance over the wavelength range of interest and a low fluorescence, e.g., a low fluorescence dye. The optical isolating agent may be added to, e.g., at least about 0.05%, at least about 0.1%, at least about 0.25%, at least about 0.5%, at least about 0.75%, or at least about 1%, or at least about 2% by weight of the mixture. The optical isolating agent may be added to, e.g., about 2% or less, about 1% or less, about 0.75% or less, about 0.5% or less, about 0.25% or less, or about 0.1% or less by weight of the mixture. The solvent may include, e.g., a hydrophilic solvent such as water or alcohol such as methanol, ethanol, or isopropyl alcohol, or combination thereof. The method may further include depositing the mixture as a layer on a surface and then drying the mixture. In some embodiments, the optical isolating agent is present in the dried layer in amount of between about 0.5% to 30% by weight. For example, the optical isolating agent may be present in the dried layer in amount of at least about 0.5%, at least about 1%, at least about 2.5%, at least about 3.5% or at least about 5% by weight of the dried layer. The optical isolating agent may be present in the dried layer in an amount of about 30% or less, about 25% or less, about 20% or less, or about 15% or less by weight of the dried layer.

In any of the foregoing embodiments, the optical isolating agent, e.g., carbon black, may be stabilized. The use of the stabilized optical isolating agents can improve rheological properties such as viscosity, sedimentation, flow and/or dispensing behavior. For example, the optical isolating agent may be stabilized using a non-ionic dispersant or slightly anionic dispersant in a concentration of 0.1% to 1% by weight of a polymer solution used to form the optical sensor. Exemplary non-ionic dispersants or slightly anionic dispersants include alcohol alkoxylates such as alcohol ethoxylates, polymeric ethoxylated non-ionic wetting agents, modified polymers with pigment affinity groups, and/or oxirane, phenyl-, polymer with oxirane, monoalkyl ethers. Exemplary commercial products include TEGO^{®} 760W, TEGO^{®} 761W, TEGO^{®} 755W, and TEGO^{®} 650. As another example, the optical isolating agent may be stabilized using a surface modified carbon black that incorporates hydrophilic groups for solvent affinity, with such stabilized carbon black present in an amount of from 0.5% to 1% by weight of a polymer solution used to form the optical sensor. Examples of such dispersants include alcohols, C13-C15, branched and/or linear, ethoxylated. Exemplary commercial products include PX Kappa and OE430W from Orion, and XSL from Kremer. As yet another example, the optical isolating agent may be stabilized using pre-stabilized carbon black dispersion from 0.5% to 1% by weight of a polymer solution used to form the optical sensor. Exemplary pre-stabilized carbon black dispersions may be stabilized with, e.g., ethylene glycol and surfactants. Suitable commercial products include AQUABLAK^{®} 8367 and AQUABLAK^{®} 8386 from Chromascape.

In examples of the disclosure, an optical sensor is prepared from a mixture including: (i) 2 to 10% by weight hydrophilic urethane polymer; (ii) 0.5% to 1% by weight stabilized optical isolating agent, e.g., stabilized carbon black; (iii) 5% by weight of any of the foregoing polymers including any of the foregoing luminescent or fluorescent reagents and, optionally, any of the foregoing optical references; and (iv) the remainder a mixture of water and a water-soluble organic solvent, e.g., a 90:10 FtOH:H2O solution. The optical sensor may be formed by a method including forming a first mixture by combining the optical isolating agent, e.g., carbon black, and the mixture of water and organic solvent. In the second step, the hydrophilic urethane polymer is added in dry form to the first mixture to form a second mixture. In the third step, the polymer including the luminescent or fluorescent reagent and optional optical reference is added to the second mixture and mixed to form a third mixture. The third mixture is applied as a thin layer within the detection zone of a microfluidic strip such as a microfluidic strip disclosed in the '946 application or '325 application. The applied mixture is dried for 3 minutes at 65 °C, in which time substantially of the solvent solution evaporates and leaves behind a single polymer layer within the detection zone of the microfluidic strip. An exemplary hydrophilic polymer is HydroMed D1, D2, D4, or combination thereof.

### BRIEF DESCRIPTION OF THE FIGURES

Fig. 1 is a side view of an optical sensor of an embodiment in accordance with the invention;
Fig. 2 is a graph of fluorescence emitted by a sodium ion luminescent or fluorescent reagent and an optical reference at different sodium ion concentrations; and
Fig. 3 is a graph of the ratio of the fluorescence of the luminescent or fluorescent reagent signal S and the optical reference signal S_{ref} obtained from the fluorescence spectra of Fig. 2.

### DETAILED DESCRIPTION

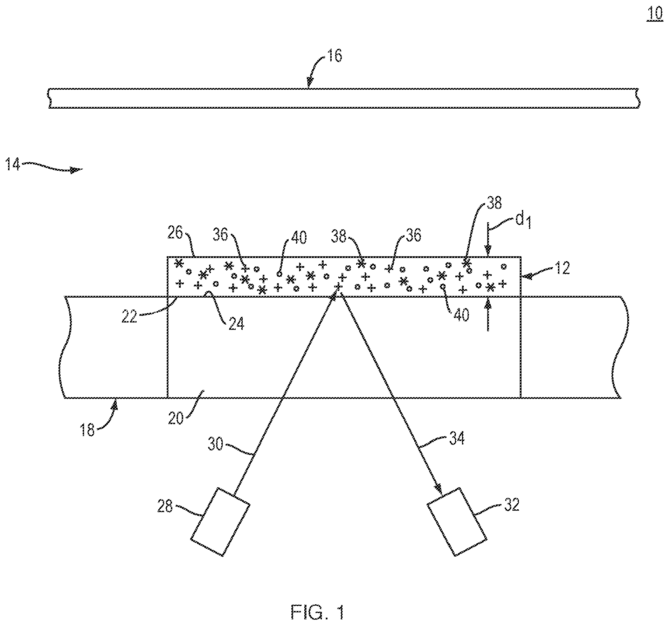

With reference to Fig. 1, an optical sensor 10 is configured to determine the presence and/or amount of a target present in a sample liquid 20. Sensor 10 includes a single polymer layer 12 disposed within a detection chamber 14 defined by walls 16 and 18. Detection chamber wall 18 includes an optically transmissive window 20. Single polymer layer 12 has a secured surface 22 secured with respect to an inner window surface 24 of optically transmissive window 20 and an exposed sensor surface 26 exposed to sample liquid within detection chamber 14. Optically transmissive window is an optically transparent support for the single polymer layer 12.

The single polymer layer 12 is a semi-permeable polymer matrix and includes, disposed therein, a luminescent or fluorescent reagent 36, an optical reference 38, and an optical isolating agent 40. Luminescent or fluorescent reagent 36 is configured to emit fluorescence when irradiated with excitation light falling within an excitation wavelength band of the luminescent or fluorescent reagent. Optical reference 38 is configured to emit fluorescence when irradiated with excitation light falling within an excitation wavelength band of the optical reference. The excitation wavelength band of luminescent or fluorescent reagent 36 and the excitation wavelength band of optical reference 38 may substantially overlap, e.g., be the same, or may have essentially no overlap so that light within the excitation wavelength band of one does not efficiently excite fluorescence from the other. The fluorescence emission range of the luminescent or fluorescent reagent 36 and optical reference 38 may substantially overlap, e.g., be the same, or may have essentially no overlap so that light detected within a fluorescence emission band of one can be distinguished from light detected within a fluorescence emission band of the other. Typically, the excitation wavelength bands of the luminescent or fluorescent reagent and optical reference are different and/or the fluorescence emission bands of the luminescent or fluorescent reagent and optical reference are different so that the reporter and reference responses can be readily distinguished.

Optical sensor 10 also includes a light source 28 and a detector 32. Light source 28 irradiates the single polymer layer 12 through window 20 with excitation light 30 falling within the excitation wavelength band of the luminescent or fluorescent reagent 36 and falling within the excitation wavelength band of the optical reference 38. Detector 32 detects fluorescence 34 through window 20 emitted from each of the irradiated luminescent or fluorescent reagent 36 and optical reference 38 residing within the single polymer layer 12. Window 20 acts as a support for single polymer layer 12 and permits the excitation and detection of luminescence from within single polymer layer 12, but window 20 does not otherwise participate in the determination of a particular target.

Optical isolating agent 40 attenuates light at wavelengths falling within the excitation wavelength band(s) and/or the fluorescence emission band(s) of the luminescent or fluorescent reagent and the optical reference. For example, the average transmittance of the single polymer layer 12 for light impinging upon the single polymer layer 12 from light source 28 within the excitation wavelength bands of one or both of luminescent or fluorescent reagent 36 and optical reference 38 may be about 50% or less, about 25% or less, about 15% or less, about 10% or less, about 5% or less, about 2% or less, or about 1% or less over a distance d1 corresponding to the thickness of the single polymer layer 12. The average transmittance of the single polymer layer 12 for light impinging upon the single polymer layer 12 from light source 28 within the excitation wavelength bands of one or both of luminescent or fluorescent reagent 36 and optical reference 38 may be at least about 0.5%, at least about 1%, at least about 2%, or at least about 3% over a distance d1 corresponding to the thickness of the single polymer layer 12. Alternatively, or in combination, the average transmittance of the single polymer layer 12 for light detected by detector 32 within the fluorescence emission bands of one or both of luminescent or fluorescent reagent 36 and optical reference 38 may be about 50% or less, about 25% or less, about 15% or less, about 10% or less, about 5% or less, about 2% or less, or about 1% or less over distance d1 corresponding to the thickness of the single polymer layer 12. The average transmittance of the single polymer layer 12 for light detected by detector 32 within the fluorescence emission bands of one or both of luminescent or fluorescent reagent 36 and optical reference 38 may be at least about 0.5%, at least about 1%, at least about 2%, or at least about 3% over a distance d1 corresponding to the thickness of the single polymer layer 12.

Because the optical isolating agent reduces the amount of excitation light that passes through the single polymer layer into the sample liquid, the amount of background fluorescence generated within the sample liquid is reduced. In addition, optical isolating agent attenuates the amount of such background fluorescence within the fluorescence emission bands of the luminescent or fluorescent reagent and optical reference that passes through the single polymer layer and reaches the detector. The optical isolating agent also attenuates some excitation light that would have otherwise reached the luminescent or fluorescent reagent and optical reference and some of the fluorescence emitted therefrom that would have otherwise reached the detector. However, because the single polymer layer is semi-permeable with respect to the target, the target diffuses through exposed sensor surface 26 into the single polymer layer where it reacts with the luminescent or fluorescent reagent. Therefore, the average pathlength within the single polymer layer for excitation light reaching the luminescent or fluorescent reagent and optical reference is shorter than the average pathlength for excitation light passing through the single polymer layer into the sample liquid. Therefore, the excitation light is attenuated to a lesser extent before reaching the luminescent or fluorescent reagent and optical reference than for the sample liquid. The average pathlength within the single polymer layer for fluorescence emitted by the luminescent or fluorescent reagent and optical reference is also shorter than the average pathlength for fluorescence emitted within the sample liquid and passing through the single polymer layer and out of the second surface 22 before being detected by detector 32. Therefore, the fluorescence emitted by the luminescent or fluorescent reagent and optical reference is attenuated to a lesser extent before reaching the detector than for fluorescence generated within the sample liquid. As a result, the background fluorescence is attenuated to a greater extent than the luminescent or fluorescent reagent fluorescence so that the signal to noise ratio is increased as compared to the absence of the carbon black.

The optical isolating agent of the single polymer layer, e.g., carbon black, may be stabilized. The use of the stabilized optical isolating agents can improve rheological properties such as viscosity, sedimentation, flow and/or dispensing behavior. For example, the optical isolating agent may be stabilized using a non-ionic dispersant or slightly anionic dispersant in a concentration of 0.1% to 1% by weight. Exemplary non-ionic dispersants or slightly anionic dispersants include alcohol alkoxylates such as alcohol ethoxylates, polymeric ethoxylated non-ionic wetting agents, modified polymers with pigment affinity groups, and/or oxirane, phenyl-, polymer with oxirane, monoalkyl ethers. Exemplary commercial products include Tego 760W, Tego 761W, Tego 755W, and Tego 650. As another example, the optical isolating agent may be stabilized using a surface modified carbon black from 0.5% to 1% by weight that incorporates hydrophilic groups for solvent affinity. Examples of such dispersants include alcohols, C13-C15, branched and/or linear, ethoxylated. Exemplary commercial products include PX Kappa and OE430W from Orion, and XSL from Kremer. As yet another example, the optical isolating agent may be stabilized using pre-stabilized carbon black dispersion from 0.5% to 1% by weight. Exemplary pre-stabilized carbon black dispersions may be stabilized with, e.g., ethylene glycol and surfactants. Suitable commercial products include aquablack 8367 and aquablack 8386 from Chromascape.

### EXAMPLES

Examples 1 and 2 refer to the following diagram:

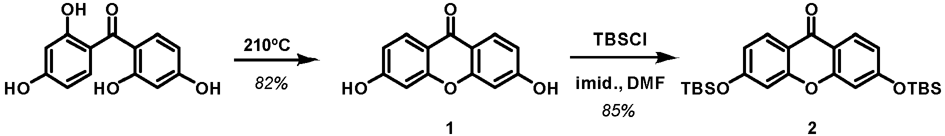

Example 1: Synthesis of 3,6-dihydroxy-9H-xanthen-9-one (1). 2,2',4,4'-Tetrahydroxybenzophenone (5.0g, 20.3mmol) was dispensed in ten equal portions into glass culture tubes sealed loosely with a rubber septum and heated to 210°C on a heat block. The reaction liquefied then re-solidified upon heating overnight. The tubes were cooled and the solids suspended in MeOH and combined. The volume was reduced to approximately 30mL by rotary evaporation and the solids were collected by filtration and rinsed with a little MeOH then dried in vacuo to give (1) (3.78g, 82%) as a brown solid. mp > 250°C. (R_{f} = 0.46, 2:8 hexanes:EtOAc). Observed [M+H]⁺ = 229.0m/z (calc'd for C₁₃H₉O₄ 229.2 m/z).

Example 2: Synthesis of 3,6-bis((tert-butyldimethylsilyl)oxy)-9H-xanthen-9-one (2). 3,6-dihydroxy-9H-xanthen-9-one (1) (2.0g, 8.76mmol) from Example 1 and imidazole (1.79g, 26.3mmol) were dissolved in anhydrous DMF (44mL) and treated with TBS-Cl (2.91g, 19.3mmol) and stirred vigorously at rt. After 4hrs, the mixture was diluted with EtOAc and washed with 1N HCl (x3), H₂O, brine, dried over Na₂SO₄, and concentrated. The residue was dissolved in CHCl₃ and purified by flash column chromatography on SiO₂ eluted with 95:5 hexanes:EtOAc. The resulting solids triturated with a small volume of hexanes, collected by filtration, and dried in vacuo to give compound **2** (3.42g, 85%) as a white crystalline solid. mp 152-153°C. (R_{f} = 0.43, 9:1 hexanes:EtOAc). ¹H NMR (400MHz, CDCl₃): δ 0.28 (s, 12H), 1.01 (s, 18H), 6.83 (s, 2H), 6.85 (dd, *J* = 9.2, 2.2 Hz, 2H), 8.20 (ddd, *J* = 9.2, 1.4, 1.4 Hz, 2H). ¹³C NMR (101MHz, CDCl₃): δ -4.21, 18.42, 25.70, 107.49, 116.57, 117.73, 128.34, 157.89, 161.51, 175.88.

Examples 3 and 4 refer to the following diagram:

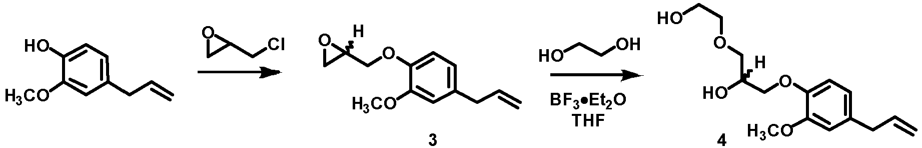

Example 3: Synthesis of (±)-2-((4-allyl-2-methoxyphenoxy)methyl)oxirane (3). Eugenol (7.4g, 45.1 mmol), ±-ephichlorohydrin (10.6mL, 135mmol), and Bu₄NHSO₄ (766mg, 2.26mmol) were dissolved in 1,4-dioxane (23mL) and treated with freshly prepared 5M NaOH (27mL) and heated in a pre-heated 80°C oil bath for 30min. The mixture was then cooled on ice and partitioned between EtOAc and sat. NaHCO₃. The aqueous was extracted once with EtOAc and the combined extract was washed with sat. NaHCO₃, brine, dried over Na₂SO₄, and concentrated. The residue was purified by flash column chromatography on SiO2 eluted with 15→20% EtOAc in hexanes to give (3) (7.5g, 75%) as a colorless oil. (R_{f} = 0.27, 8:2 hexanes:EtOAc). ¹H NMR (400MHz, CDCl₃): δ 2.73 (dd, *J* = 4.8, 2.7 Hz, 1H), 2.89 (dd, *J* = 4.8, 4.2 Hz, 1H), 3.33 (br d, *J* = 6.7 Hz, 1H), 3.36-3.41 (m, 1H), 3.38 (s, 3H), 4.02 (dd, *J* = 11.4, 5.5 Hz, 1H), 4.21 (dd, *J* = 11.4, 3.6 Hz, 1H), 5.04-5.07 (m, 1H), 5.07-5.11 (m, 1H), 5.95 (dddd, *J* = 16.9, 10.2, 6.7, 6.7 Hz, 1H), 6.69-6.74 (m, 2H), 6.87 (d, *J* = 8.1 Hz, 1H).

Example 4: Synthesis of (±)-1-(4-allyl-2-methoxyphenoxy)-3-(2-hydroxyethoxy)propan-2-ol (4). (±)-2-((4-allyl-2-methoxyphenoxy)methyl)oxirane from Example 3 (7.5g, 34mmol) was dissolved in anhydrous THF (34mL) and anhydrous ethylene glycol (34mL) and cooled to 0°C then treated with BF₃•Et₂O. The mixture was stirred for 30min, then the cooling bath was removed and stirring continued for an additional 5 hrs. The mixture was diluted with EtOAc and washed with sat. NaHCO₃ (x3), brine (x2), dried over Na₂SO₄, and concentrated. The residue was purified by flash column chromatography on SiO₂ eluted with 1→5% MeOH in CH₂Cl₂ to give (4) (7.47g, 78%) as a colorless, viscous oil. (R_{f} = 0.43, 9:1 hexanes:EtOAc). ¹H NMR (400MHz, CDCl₃): δ 3.30 (br s, 2H), 3.33 (br d, J = 6.68 Hz, 2H), 3.60-3.63 9m, 2H), 3.65 (d, J = 5.8 Hz, 1H), 3.69 (dd, J = 10.1, 4.0 Hz, 1H), 3.72-3.76 (m, 1H), 3.84 (s, 3H), 4.02 (dd, J = 9.8, 6.5 Hz, 1H), 4.05 (dd, J = 9.6, 4.5 Hz, 1H), 4.15-4.22 (m, 1H), 5.04-5.06 (m, 1H), 5.06-5.10 (m, 1H), 5.94 (dddd, J = 16.9, 10.2, 6.7, 6.7 Hz, 1H), 6.69-6.73 (m, 2), 6.85 (d, J = 8.6 Hz, 1H).

Examples 5-9 refer to the following diagram:

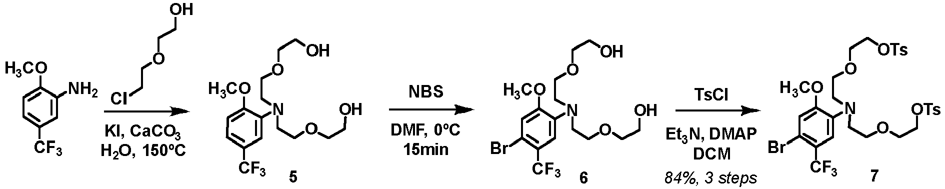

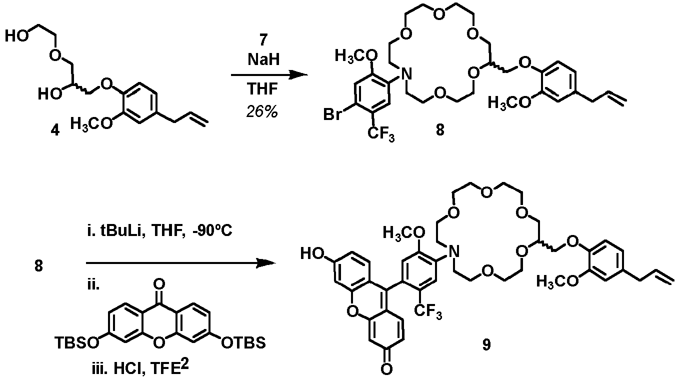

Example 5: 2,2'-((((2-methoxy-5-(trifluoromethyl)phenyl)azanediyl)bis(ethane-2,1-diyl))bis(oxy))bis(ethan-1-ol) (5). A 150mL pressure bottle was charged with 2-methoxy-5-(trifluoromethyl)aniline (972mg, 5.09 mmol. *Caution: stench chemical*.), KI (1.27g, 7.64mmol), CaCO₃ (560mg, 5.60mmol), and 2-(2-chloroethoxy)ethanol (1.27mL, 7.64mmol) and heated to 150°C under N₂ atmosphere overnight. The reaction was cooled, vented, and additional CaCO₃ (1.0g) and 2-(2-chloroethoxy)ethanol (2mL) was introduced and the mixture re-heated to 150°C. After 6hrs, the mixture was cooled and diluted with 1N HCl then washed with Et₂O (x2). The combined wash was back-extracted with 1N HCl once and the combined aqueous was basified with K₂CO₃, shaken with EtOAc, and filtered through Celite. The organic phase was collected and the aqueous extracted twice more with EtOAc. The combined extract was washed with brine, dried over Na₂SO₄, and concentrated to give (5) (2.3g, >100%) of an orange colored oil which was used without further purification.

Example 6: 2,2'-((((4-bromo-2-methoxy-5-(trifluoromethyl)phenyl)azanediyl)bis(ethane-2,1-diyl))bis(oxy))bis(ethan-1-ol) (6). The aniline compound (5) from Example 5 (2.3g crude, 5.09mmol) was dissolved in DMF, cooled to 0°C, and treated with solid N-bromosuccinimide (906mg, 5.09mmol). After 5 min, additional NBS (75mg) was added. After an additional 5min, the reaction mixture was poured into 1N HCl (75mL) and washed with Et₂O (2x50mL). The combined wash was back-extracted with 1N HCl (25mL) and the combined aqueous was neutralized with K₂CO₃ and extracted with EtOAc (x3). The combined extract was washed with brine (x2), dried over Na₂SO₄, and concentrated to give (6) as a brown oil which was used without further purification.

Example 7: ((((4-bromo-2-methoxy-5-(trifluoromethyl)phenyl)azanediyl)bis(ethane-2,1-diyl))bis(oxy))bis(ethane-2,1-diyl) bis(4-methylbenzenesulfonate) (7). The diol compound (6) from Example 6 (2.5g crude, 5.09mmol) was dissolved in anhydrous DCM (10mL), cooled to 0°C, and treated with tosyl chloride (2.13g, 11.2mmol), Et₃N (1.77mL, 12.7mmol), and DMAP (62mg, 0.51mmol). The cooling bath was removed and the mixture was stirred at rt for 75min then diluted with EtOAc and washed with sat. NaHCO₃ (x3), brine, dried over Na₂SO₄, and concentrated. The residue was purified by flash column chromatography on SiO₂ eluted with 20→40% EtOAc in hexanes to give (7) (3.21g, 84% over 3 steps) as a faintly yellow oil. (R_{f} = 0.39, 6:4 hexanes:EtOAc). Observed [M+H]⁺ = 754.3/756.3 m/z (1:1) (calc'd for C₃₀H₃₆BrF₃NO₉S₂ 754.1 m/z).

Example 8: 5-((4-allyl-2-methoxyphenoxy)methyl)-16-(4-bromo-2-methoxy-5-(trifluoromethyl)phenyl)-1,4,7,10,13-pentaoxa-16-azacyclooctadecane (8). The 1,5-diol compound (4) from Example 4 (1.20g, 4.25mmol) and the ditosylate compound (7) from Example 7 (3.21g, 4.25mmol) were combined and co-evaporated once from anhydrous THF, dried briefly in vacuo, then redissolved in anhydrous THF (10mL). A 100mL round bottom flask fitted with a reflux condenser was flame dried then charged with anhydrous THF (30mL), and NaH (1.02g, 25.5mmol) that had been washed twice with hexanes immediately prior. The mixture was heated to reflux and the solution of compounds (4) and (7) was added dropwise over a period of 30min. After an additional 4 hrs, the mixture was cooled to rt and carefully poured into a mixture of EtOAc and sat. NH₄Cl. The organic phase was collected and the aqueous extracted twice more with EtOAc. The combined extract was dried over MgSO₄, amended with 5%vol MeOH, and filtered through a pad of basic alumina. The filtrate was concentrated and purified by flash column chromatography on SiO₂ eluted with 20→50% EtOAc in hexanes + 2%vol Et₃N to give (8) (777mg, 26%) as a faintly fellow oil. ¹H NMR (400MHz, CDCl₃): δ 3.32 (br d, J = 6.6 Hz, 2H), 3.46 (br dd, J = 12.5, 6.1 Hz, 4H), 3.52-3.80 (m, 18H), 3.82 (s, 3H), 3.85 (s, 3H), 3.96-4.04 (m, 1H), 4.04-4.10 (m, 2H), 5.94 (dddd, J = 16.8, 10.1, 6.7, 6.7 Hz, 1H), 6.66-6.72 (m, 2H), 6.82-6.89 (m, 1H), 7.05 (s, 1H), 7.32 (s, 1H). ¹³C NMR (101MHz, CDCl₃): δ 39.9, 52.2, 52.5, 56.06, 56.07, 69.7, 69.8, 69.9, 70.0, 70.3, 70.7, 70.9, 71.10, 71.11, 71.7, 78.0, 109.4, 111.6, 112.7, 114.3, 115.7, 117.6, 118.8, 119.6, 119.7, 120.7, 122.1, 122.4, 124.1, 124.8, 130.7, 133.5, 137.8, 139.1, 146.9, 149.7, 155.0. ¹⁹F NMR (101MHz, CDCl₃): δ -61.0. Observed [M+H]⁺ = 692.4/694.4 m/z (1:1) (calc'd for C₃₁H₄₂BrF₃NO₈ 692.2 m/z).

Example 9: 9-(4-(5-((4-allyl-2-methoxyphenoxy)methyl)-1,4,7,10,13-pentaoxa-16-azacyclooctadecan-16-yl)-5-methoxy-2-(trifluoromethyl)phenyl)-6-hydroxy-3H-xanthen-3-one (9). The aryl bromide compound (8) of Example 8 (150mg, 0.217mmol) was dissolved in MeOH and filtered through a pad of basic alumina and the filtrate was concentrated, co-evaporated twice from 2-Me-THF, and dried thoroughly in vacuo. The residue was redissolved in anhydrous 2-Me-THF (1.1mL) and cooled in to -116°C (N₂/EtOH) and *tert*-butyllithium (285µL, 0.541mmol, 1.9M in pentane) was added via the cold sidewall. The pale orange suspension was stirred cold for 10min. Xanthone 2 (119mg, 0.26mmol) was dried by gently warming in vacuo then dissolved in anhydrous 2-Me-THF (1mL) and added slowly to the aryllithium intermediate via the cold sidewall. A dark red color developed. The mixture was stirred cold for 10min, then the cooling bath was removed and the mixture was stirred at rt for 10min which caused the color to fade to orange. A mixture of TFE (1.1mL) and 2N HCl (1.1mL) was added which caused the color to dissipate, liberating an intermediate green color, and ultimately a bright red/orange color. The mixture was stirred for 10min then diluted with 0.5M HCl and washed with Et₂O (x2). The ethereal wash was back-extracted with two small portions of 0.5M HCl and the combined aqueous was neutralized with Na₂CO₃ then back-extracted with EtOAc (x3). The extract was dried over MgSO₄ and concentrated, and the residue was purified by flash column chromatography on SiO₂ eluted with 2→12% MeOH in CH₂Cl₂ + 2%vol Et₃N to give (9) (59.4mg, 33%) as an orange film. Observed [M+H]⁺ = 824.2 m/z (calc'd for C₄₄H₄₉F₃NO₁₁ 824.3 m/z).

Example 10: Preparation of a sodium ion (Na⁺) single polymer layer

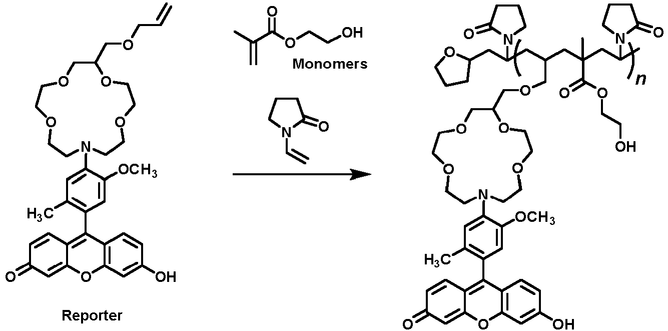

A luminescent or fluorescent reagent having a terminal vinyl group is reacted with two vinyl monomers in a solution of 2% w/v azobisisobutyronitrile (AIBN) in THF at 70 °C. The resulting hydrophilic polymer between about 0.1% to 1% by weight luminescent or fluorescent reagent, which is covalently bound to the polymer. The polymer is semipermeable with respect to sodium ion.

Example 11: Preparation of an optical sensor for the determination of sodium ion: A mixture is prepared containing: (i) 10% by weight hydrophilic urethane polymer; (ii) 1% by weight carbon black; (iii) 5% by weight of the polymer of Example 10; and (iv) the remainder a 90:10 FtOH:H2O solution. The polymer is applied as a thin layer within the detection zone of a microfluidic strip such as a microfluidic strip disclosed in the '946 application or '325 application. The applied polymer is dried for 3 minutes at 65 °C, in which time substantially of the solvent solution evaporates and leaves behind a single polymer layer within the detection zone of the microfluidic strip.

In use, the microfluidic strip is inserted into a reader, such as one described in the '946 application or '325 application. A liquid sample, e.g., of blood, is applied to the strip. The reader operates the strip to introduce the sample into the detection zone whereupon sodium ion present in the sample diffuses into the single polymer layer therein and interacts with the luminescent or fluorescent reagent residing within the layer. The reader has a light source that irradiates the single polymer layer with excitation light causing the luminescent or fluorescent reagent to emit fluorescence indicative of the presence and/or amount of sodium ion present in the liquid sample. The carbon black attenuates the excitation light passing through the single polymer layer into the sample liquid within the detection zone thereby reducing the amount of background fluorescence generated from the sample liquid. In addition, the carbon black attenuates the amount of background fluorescence that is generated before it passes through the single polymer layer and reaches the detector of the reader. The carbon black also attenuates some excitation light before it reaches the luminescent or fluorescent reagent and some of the fluorescence emitted by the luminescent or fluorescent reagent. However, because the sodium ion diffuses into the single polymer layer, the average pathlength within the single polymer layer of such excitation light and fluorescence is less than average pathlength for light traveling through the single polymer layer into the liquid and for background fluorescence emitted within the liquid and traveling through the single polymer layer out of the detection zone. Therefore, the background fluorescence is attenuated to a greater extent than the luminescent or fluorescent reagent fluorescence so that the signal to noise ratio is increased as compared to the absence of the carbon black.

Example 12: Preparation of a sodium ion (Na⁺) single polymer layer including a luminescent or fluorescent reagent and an optical reference

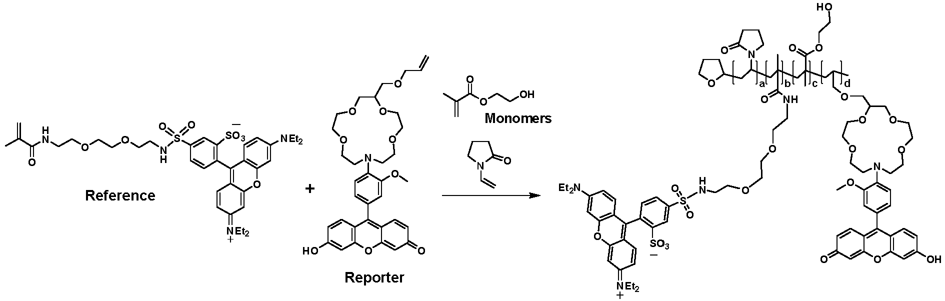

A luminescent or fluorescent reagent and an optical reference each having a terminal vinyl group are reacted with two vinyl monomers in a solution of 2% w/v azobisisobutyronitrile (AIBN) in THF at 70 °C. The resulting hydrophilic polymer between about 0.1% to 1% by weight each of the luminescent or fluorescent reagent and optical reference, which are covalently bound to the polymer. The polymer is semipermeable with respect to sodium ion.

Example 13: Preparation of an optical sensor with internal reference for the determination of sodium ion. A mixture is prepared containing: (i) 10% by weight hydrophilic urethane polymer; (ii) 1% by weight carbon black; (iii) 5% by weight of the polymer of Example 12; and (iv) the remainder a 90:10 FtOH:H2O solution. The polymer is applied as a thin layer within the detection zone of a microfluidic strip such as a microfluidic strip disclosed in the '946 application or '325 application. The applied polymer is dried for 3 minutes at 65 °C, in which time substantially of the solvent solution evaporates and leaves behind a single polymer layer within the detection zone of the microfluidic strip.

Example 14: Calibration spectra and calibration curve for a sodium sensor.

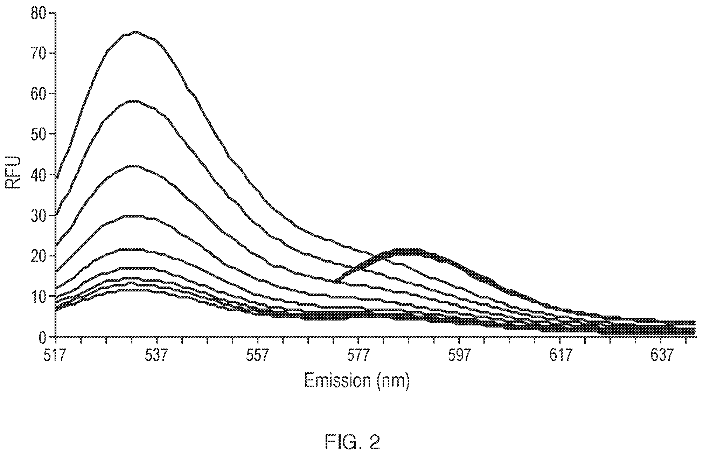

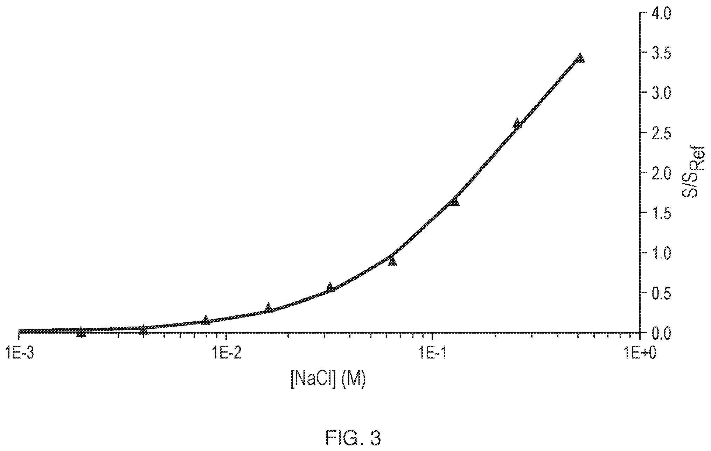

The mixture of Example 13 was applied to a surface of an optical flow cell and dried as per Example 13. The flow cell was inserted into a fluorimeter configure to irradiate the dried single polymer layer with excitation light at 490 nm to excite fluorescence from the luminescent or fluorescent reagent and with excitation light at 550 nm to excite fluorescence from the optical reference. The fluorescence with each excitation light was measured upon exposing the single polymer layer to solutions containing different sodium ion concentrations (Fig. 2). The luminescent or fluorescent reagent emits a fluorescence signal "S" indicative of the presence and/or amount of sodium ion present in the liquid sample. The excitation light causes the optical reference to emit a fluorescence signal "S_{Ref}" the intensity and amount of which is insensitive to the presence and/or amount of sodium ion or concomitant species present in the sample liquid. Accordingly, the luminescent or fluorescent reagent fluorescence can be normalized by the optical reference fluorescence (S/S_{ref}) to correct for variations in the system thereby increasing the accuracy and precision of the sodium ion determination (Fig. 3). The carbon black attenuates the signals, including the background fluorescence and optical reference and luminescent or fluorescent reagent fluorescence increasing the signal to noise ratio.

Examples 15 to 25 Refer to the following diagram:

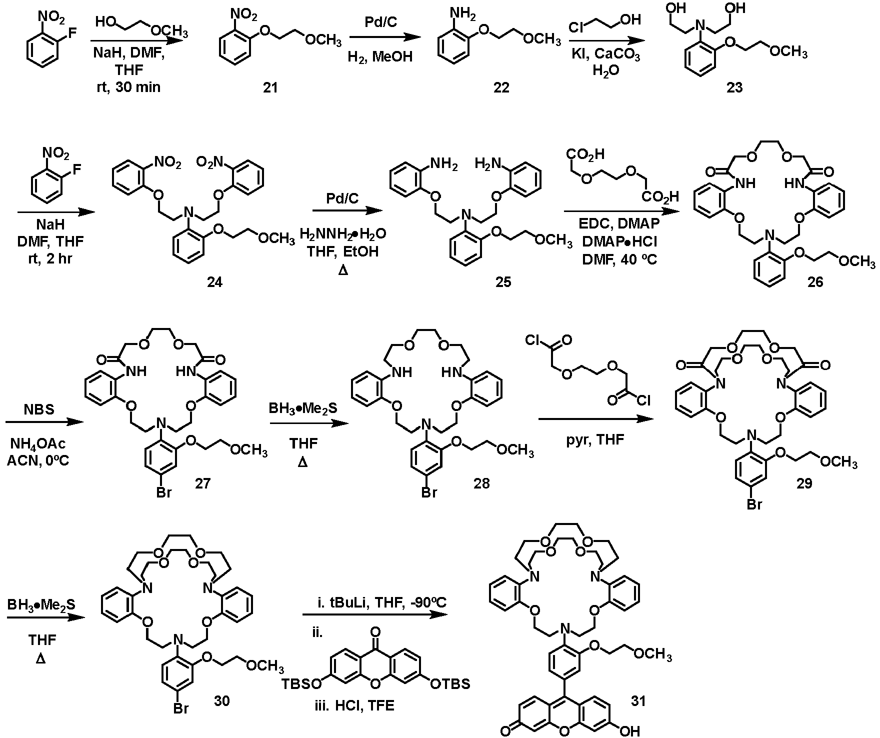

Example 15: 1-(2-methoxyethoxy)-2-nitrobenzene (21). 1-Fluoro-2-nitrobenzene (10g, 71mmol) and 2-methoxyethanol (6.5g, 85mmol) were dissolved in DMF:THF (30:1, 70mL) and NaH dispersion (3.4g, 85mmol) was added portionwise over approximately 15min. After an additional 15min, the reaction was quenched by careful addition of H₂O then diluted with EtOAc and washed with H₂O (x1), K₂CO₃ (x2), brine, dried over Na₂SO₄, and concentrated. The residue was taken up in MeOH and washed with three small portions of hexanes. The methanolic phase was concentrated and distilled in vacuo to give compound (21) (11.1g, 79%) as an orange oil. Bp 120-130°C, 133.32 Pa (1 torr). (R_{f} = 0.15, 8:2 hexanes:EtOAc). ¹H NMR (400MHz, CDCl₃): δ 3.45 (s, 3H), 3.78-3.82 (m, 2H), 4.23-4.28 (m, 2H), 7.04 (ddd, J = 8.1, 7.4, 1.0 Hz, 1H), 7.11 (dd, J = 8.5, 1.0Hz, 1H), 7.52 (ddd, J = 8.5, 7.4, 1.7Hz, 1H), 7.83 (dd, J = 8.1, 1.7Hz, 1H).

Example 16: 2-(2-methoxyethoxy)aniline (22). Nitro compound 21 (11.1g, 56.1mmol) was dissolved in MeOH (140mL). A slurry of Pd/C in MeOH was prepared under N₂ atmosphere and added to the substrate solution. The vessel was evacuated and backfilled with H₂ several times then stirred vigorously for 21hrs. The mixture was filtered through Celite and the cake carefully rinsed with MeOH. The filtrate was concentrated and purified by flash column chromatography on SiO₂ eluted with 20→40% EtOAc in hexanes to give compound (22) (8.48g, 90%) as a pale brown oil. (R_{f} = 0.38, 6:4 hexanes:EtOAc). ¹H NMR (400MHz, CDCl₃): δ 3.45 (s, 3H), 3.74-3.78 (m, 2H), 3.86 (br s, 2H), 4.12-4.17 (m, 2H), 6.69-6.77 (m, 2H), 6.79-6.84 (m, 2H). Observed [M+H]⁺ = 168.3 m/z (calc'd for C₉H₁₄NO₂ 168.1 m/z).

Example 17: 2,2'-((2-(2-methoxyethoxy)phenyl)azanediyl)bis(ethan-1-ol) (23). A 150mL pressure bottle was charged with aniline **22** (8.48g, 50.7mmol), KI (8.42g, 50.7mmol), CaCO₃ (45.6mmol), and 2-chloroethanol (10.2mL, 152mmol) and the apparatus was purged with N₂, sealed, and heated to 110°C for 20hr. The mixture was diluted with 1N HCl and washed with two small volumes of Et₂O. The combined wash was back-extracted with 1N HCl once. The combined aqueous phase was basified with conc. NH₄OH at 0°C and extracted with CH₂Cl₂ (x5). The combined extract was washed with 1M NaOH, brine, dried over Na₂SO₄, then diluted with 0.1 volumes of MeOH filtered through a thin pad of SiO₂ rinsing with 9:1 CH₂Cl₂:MeOH. The filtrate was concentrated to give compound (23) (12.3g, 95%) as a pale brown oil. ¹H NMR (400MHz, CDCl₃): δ 3.14-3.18 (m, 4H), 3.44 (s, 3H), 3.45-3.51 (m, 4H), 3.61 (br s, 2H), 3.74-3.78 (m, 2H), 4.11-4.15 (m, 2H), 6.92 (dd, J = 8.2, 1.4 Hz, 1H), 6.99 (ddd, J = 7.6, 7.6, 1.4 Hz, 1H), 7.13 (ddd, J = 8.1, 7.5, 1.7 Hz, 1H), 7.22 (dd, J = 7.8, 1.7Hz, 1H). Observed [M+H]⁺ = 256.2 m/z (calc'd for C₁₃H₂₂NO₄ 256.1 m/z).

Example 18: 2-(2-methoxyethoxy)-*N*,*N*-bis(2-(2-nitrophenoxy)ethylaniline (24). Compound 23 (5.49g, 21.5mmol) and 1-fluoro-2-nitrobenzene were dissolved in anhydrous DMF:THF (30:1, 27mL) and NaH dispersion (1.89g, 47.3mmol) was added portionwise over a period of approximately 15min. An exotherm resulted which was not mitigated. After 2hrs, the reaction was carefully quenched with H₂O then partitioned between EtOAc and H₂O and the aqueous phase extracted twice more with EtOAc. The combined extract was washed with 5% K₂CO₃ (x3), brine, dried over Na₂SO₄, and concentrated. The residue was purified by flash column chromatography on SiO₂ eluted with 30→40% EtOAc in hexanes to give compound (24) (9.9g, 92%) as a yellow colored oil that crystallized upon standing. Observed [M+H]⁺ = 498.2 m/z (calc'd for C₂₅H₂₈N₃O₈ 498.2 m/z).

Example 19: N,N-bis(2-(2-aminophenoxy)ethyl)-2-(2-methoxyethoxy)aniline (25). bis-Nitro compound 24 (4.98g, 10mmol) was dissolved in THF:EtOH (1:1, 50mL). A slurry of Pd/C in EtOH was prepared under N₂ atmosphere and added to the substrate solution. The mixture was warmed to 50°C and hydrazine hydrate 4.9mL was added dropwise, addition of which initially caused effervescence. Following addition, the mixture was warmed to gentle reflux for 12hrs then cooled to rt and filtered through Celite carefully rinsing with THF. The filtrate was concentrated and the residue was treated with 5% Na₂CO₃ (50mL) and extracted with EtOAc (3x30mL). The combined extract was washed with brine, dried over Na₂SO₄, and concentrated. The residue was purified by flash column chromatography on SiO₂ eluted with 50% EtOAc in hexanes to give compound (25) (3.35g, 76%) as a faintly brown colored viscous oil. (R_{f} = 0.31, 1:1 hexanes:EtOAc). ¹H NMR (400MHz, CDCl₃): δ 3.39 (s, 3H), 3.70-3.76 (m, 6H), 4.09-4.16 (m, 6H), 6.63-6.70 (m, 4H), 6.72-6.80 (m, 4H), 6.87-6.92 (m, 2H), 6.94-7.00 (m, 1H), 7.08-7.12 (m, 1H). Observed [M+H]⁺ = 438.2 m/z (calc'd for C₂₅H₃₂N₃O₄ 438.2 m/z).

Example 20: 22-(2-(2-methoxyethoxy)phenyl)-9,10,21,22,23,24-hexahydro-5H,12H,20H-dibenzo[h,q][1,4,10,16]tetraoxa[7,13,19]triazacyclohenicosine-6,13(7H,14H)-dione (26). bis-Aniline compound 25 (3.35g, 7.66mmol) and 3,6-dioxa-1,8-dioic acid were dissolved in anhydrous DMF (20mL) and added via syringe pump over a period of 90min to a stirred suspension of EDC•HCl (7.34g, 38.3mmol), DMAP (936mg, 7.66mmol), and KBF₄ (964mg, 7.66mmol) in anhydrous DMF (130mL) at 40°C. The mixture was stirred for 12hr then concentrated to approximately one third the initial volume and partitioned between EtOAc and half-saturated NaHCO₃. The aqueous phase was extracted with EtOAc and the combined extract was washed with sat. NaHCO₃ (x2), brine, dried over Na₂SO₄, and concentrated. The residue was purified by flash column chromatography on SiO₂ eluted with 1→3% MeOH in CH₂Cl₂ to give compound (26) (2.51g, 57%) as a pale yellow oil that solidified upon standing. (R_{f} = 0.58, 94:6 CHCl₃:MeOH). ¹H NMR (400MHz, CDCl₃): δ 3.41 (s, 3H), 3.70-3.74 (m, 2H), 3.84 (t, J = 5.7 Hz, 4H), 3.93 (s, 4H), 4.09 (t, J = 5.7 Hz, 4H), 4.08-4.12 (m, 2H), 4.15 (s, 4H), 6.75 (d, J = 7.8 Hz, 1H), 6.76 (d, J = 7.9 Hz, 1H), 6.86-6.91 (m, 2H), 6.91-7.01 (m, 5H), 7.03-7.07 (m, 1H), 8.35 (dd, J = 7.6, 1.8 Hz, 1H), 9.20 (br s, 1H). Observed [M+H]⁺ = 580.3 m/z (calc'd for C₃₁H₃₈N₃O₈ 580.3 m/z).

Example 21: 22-(4-bromo-2-(2-methoxyethoxy)phenyl)-9,10,21,22,23,24-hexahydro-5H,12H,20H-dibenzo[h,q][1,4,10,16]tetraoxa[7,13,19]triazacyclohenicosine-6,13(7H,14H)-dione (27). Aniline compound 26 (2.47g, 3.75mmol) was dissolved in DMF (21mL) and treated with NBS (741mg, 4. 16mmol) with vigorous stirring at rt. After 10min, additional NBS (82mg, 463µmol) was added. After an additional 10min, the reaction mixture was diluted with EtOAc and washed with Na₂CO₃ (x2), brine, dried over Na₂SO₄, and concentrated. The residue was purified by flash column chromatography on SiO₂ eluted with 0.5→3% MeOH in CH₂Cl₂ to give compound 27 (2.97g, greater than theory) as a brown oil which crystallized upon standing. (R_{f} = 0.37, 99:1 CHCl₃:MeOH). Observed [M+H]⁺ = 658.4/660.4 m/z (1:1) (calc'd for C₃₁H₃₇BrN₃O₈ 658.2 m/z).

Example 22: 22-(4-bromo-2-(2-methoxyethoxy)phenyl)-6,7,9,10,13,14,21,22,23,24-decahydro-5H,12H,20H-dibenzo[h,q][1,4,10,16]tetraoxa[7,13,19]triazacyclohenicosine (28). bis-Amide compound 27 (2.97g, approx. 4.26mmol) was dissolved in anhydrous THF (43mL) and treated with BH₃•Me₂S and the mixture was heated to gentle reflux under N₂ atmosphere for 14hrs. The mixture was then cooled and quenched by dropwise addition of 3N HCl (21mL) then returned to reflux for 30 min and again cooled. The solution was rendered basic by the addition of 10M NaOH and extracted with EtOAc (x3). The combined extract was washed with brine (x2), dried over Na₂SO₄, and concentrated. The residue was purified by flash column chromatography on SiO₂ eluted with 30→50% EtOAc in hexanes to give compound 28 (2.33g, 87%) as a faintly yellow heavy oil. (R_{f} = 0.21, 7:3 hexanes:EtOAc). Observed [M+H]⁺ = 630.2/632.3 m/z (1:1) (calc'd for C₃₁H₄₁BrN₃O₆ 630.2 m/z).

Example 23: Dioxo-2.2.3-cryptand (29). bis-Aniline compound 28 (2.33g, 3.69mmol) and anhydrous pyridine (654µL, 8.12mmol) were dissolved in anhydrous DCM (10mL). Separately, 3,6-dioxaoctan-1,8-dioic acid chloride (873mg, 4.06mmol) was dissolved in anhydrous DCM (10mL). These two solutions were added via syringe to a 250mL round bottom flask containing anhydrous THF (74ml) over a period of approximately 6 hours. The turbid mixture was stirred at rt overnight then diluted with EtOAc and washed with sat NaHCO₃ (x3), brine, dried over MgSO₄, and concentrated. The residue was purified by flash chromatography on SiO₂ eluted with 0→1% MeOH in CH₂Cl₂ with 1% Et₃N to give compound 29 (1.2 g, 42%) as a white foam. Observed [M+H]⁺ = 772.3/774.3 m/z (1:1) (calc'd for C₃₇H₄₇BrN₃O₁₀ 772.2 m/z).

Example 24: 2.2.3-cryptand (30). Diamide compound 29 (1.2g, 1.55mmol) was dissolved in anhydrous THF (16 mL) and BH₃•Me₂S (15.5mmol, 1.47 mL) was added via syringe. The mixture was warmed to 60 °C and stirred for 2 hours. A white precipitate was observed after 1 hour. The reaction was cooled to 0 °C and carefully quenched by dropwise addition of 1N HCl (25 mL) then re-heated to 60 °C for 30 min. The reaction was again cooled to 0 °C and the pH adjusted above 10 by addition of solid NaOH. The mixture was extracted with CHCl₃ (x3), and the combined extract was washed with brine, dried over Na₂SO4 and concentrated. The residue was dissolved in DCM and purified by flash chromatography on SiO₂ eluted with 0→1% MeOH in DCM and 1% Et₃N to give 30 (1.1 g, 95%) as a foam. Observed [M+H]⁺ = 743.9/746.0 m/z (1:1) (calc'd for C₃₇H₅₁BrN₃O₈ 743.3 m/z).

Example 25: 2.2.3-cryptand K⁺ sensor B (31). Bromide compound 30 (0.208mmol, 155 mg) was dissolved in DCM:MeOH (9:1) and filtered through a pipet column of basic alumina then concentrated and further dried in vacuo, The residue was co-evaporated twice from anhydrous 2-methyl-THF then dried in vacuo. The residue was redissolved in anhydrous 2-Me-THF (1.0mL) and cooled in to -116 °C (N₂/EtOH) and *tert*-butyllithium (275µL, 0.52mmol, 1.9M in pentane) was added via the cold sidewall. The suspension was stirred cold for 10min. Xanthone (116mg, 0.250mmol) was dried by gently warming in vacuo then dissolved in anhydrous 2-Me-THF (1mL) and added slowly to the aryllithium intermediate via the cold sidewall. A dark red color developed. The mixture was stirred cold for 15min, then the cooling bath was removed and the mixture was stirred at rt for 20min. A mixture of TFE (1mL) and 2N HCl (1mL) was added. The mixture was stirred for 10min then diluted with 0.5M HCl and washed with 1:1 EtOAc:Hexanes. The aqueous was adjusted to pH 4 and back-extracted with EtOAc (x5). The extract was purified by prep HPCL (10→100% ACN+0.1% HCO₂H over 40min, 20ml/min, Clipeus C18 20x250mm, 10µm) to give compound 31 (88mg, 48%) as a green film. Observed [M+H]⁺ = 876.6 m/z (calc'd for C₅₀H₅₈N₃O₁₁ 876.4 m/z).

### EQUIVALENTS

The foregoing embodiments are therefore to be considered in all respects illustrative rather than limiting on the invention described herein. Scope of the invention is thus indicated by the appended claims rather than by the foregoing description, and all changes that come within the meaning of the claims are intended to be embraced therein.

## Claims

1. An optical sensor (10) for detecting a target present in a sample fluid (20), comprising:
a detection chamber (14) configured to receive the sample fluid (20);
a single polymer layer (12) disposed in the detection chamber (14), the single polymer layer (12) comprising:
i) a luminescent or fluorescent reagent (36) that is covalently bound to a polymer of the single polymer layer (12) and is configured to generate a luminescent or fluorescent reagent signal indicative of the presence of the target within the single polymer layer (12) when the single polymer layer (12) is irradiated with excitation light (30) from an excitation source (28), ii) an optical isolating agent (40), and iii) an optical reference (38) that is covalently bound to a polymer of the single polymer layer (12) and is configured to generate an optical reference signal generally independent of the presence of the target within the single polymer layer (12) when the single polymer layer (12) is irradiated with the excitation light (30) from the excitation source (28), and wherein the single polymer layer (12) iv) is at least semi-permeable to the target, has a first surface (26) configured to contact sample fluid (20) present in the detection chamber (14), vi) has a second surface (22) secured to a support that is optically transparent at wavelengths useful for exciting and detecting luminescence or fluorescence from the luminescent or fluorescent reagent (36) and/or the optical reference (38) present therein, and vii) has a transmittance of about 20% or less at a peak excitation wavelength of the luminescent or fluorescent reagent (36) along an optical axis that is normal to the second surface (22) of the single polymer layer (12).

2. The optical sensor of claim 1, wherein the single polymer layer has a thickness in the dry state, of about 75 µm or less along the optical axis normal to the second surface (22) of the single polymer layer (12).

3. The optical sensor (10) of claim 1 or 2, wherein the single polymer layer (12) has a thickness in the dry state, of at least about 20 µm along the optical axis normal to the second surface (22) of the single polymer layer (12).

4. The optical sensor (10) of any of the foregoing claims, wherein the detection chamber (14) comprises a first wall (18) that is transparent at the peak excitation wavelength of the luminescent or fluorescent reagent (36), the second surface (22) of the single polymer layer (12) is disposed against an inner surface of the first wall (18) of the detection chamber (14), and the optical axis normal to the second surface (22) passes through the first wall (18) into the single polymer layer (12).

5. The optical sensor (10) of any of the foregoing claims, wherein the single polymer layer (12) comprises carbon black, carbon nanostructures, and/or a non-fluorescent dye.

6. The optical sensor (10) of any of the foregoing claims, wherein the sample fluid (20) comprises blood, plasma, or serum.

7. The optical sensor (10) of any of the foregoing claims wherein the target is selected from a group consisting of Ca⁺⁺, K⁺, Na⁺, or H⁺, creatinine, lactate, and glucose.

8. A method of detecting a target present in a sample fluid (20), the method comprising:
contacting a first surface (26) of a single polymer layer (12) with the sample fluid (20), the single polymer layer (12) being at least semi-permeable to the target and comprising i) a luminescent or fluorescent reagent (36) covalently bound to a polymer of the single polymer layer (12) and configured to generate a luminescent or fluorescent reagent signal indicative of the presence of the target within the single polymer layer (12) when the single polymer layer (12) is irradiated with excitation light (30) from an excitation source (28), ii) an optical isolating agent (40) and iii) an optical reference (38) covalently bound to a polymer of the single polymer layer (12) and configured to generate an optical reference signal generally independent of the presence of the target within the sample fluid (20) when the single polymer layer (12) is irradiated with excitation light (30) from the excitation source (28),
irradiating the single polymer layer (12) with the excitation light (30) along an optical axis that passes into the single polymer layer (12) through a second surface (22) of the single polymer layer (12) not in contact with the sample fluid, the optical axis being normal to the second surface (22) of the single polymer layer (12), wherein the second surface (22) is secured to a support that is optically transparent at wavelengths useful for exciting and detecting luminescence or fluorescence from a luminescent or fluorescent reagent (36) and/or an optical reference (38) present therein, and
the single polymer layer (12) transmitting about 20% or less at a peak excitation wavelength of the luminescent or fluorescent reagent (36) along the optical axis

9. The method of claim 8, wherein essentially all of the detected fluorescence arises from the luminescent or fluorescent reagent (36) or the optical reference (38) in the single polymer layer (12).

10. The method of claim 8 or 9, wherein the target is selected from a group consisting of Ca⁺⁺, K⁺, Na⁺, or H*⁺*, creatinine, lactate, and glucose.

11. The method of any of claims 8-10, wherein the single polymer layer (12) comprises carbon black, carbon nanostructures, and/or a non-fluorescent.

12. The method of any of claims 8-11, wherein the single polymer layer (12) has a thickness, in the dry state, of about 75 µm or less.

13. The method of any of claims 8-12, wherein the single polymer layer (12) has a thickness, in the dry state, of at least about 20 µm.

## Patentansprüche

1. Optischer Sensor (10) zum Detektieren eines in einem Probenfluid (20) vorhandenen Zielobjekts, umfassend:
eine Detektionskammer (14), die zum Aufnehmen des Probenfluids (20) konfiguriert ist;
eine Einzelpolymerschicht (12), die in der Detektionskammer (14) angeordnet ist, wobei die Einzelpolymerschicht (12) umfasst:
i) ein lumineszierendes oder fluoreszierendes Reagenz (36), das kovalent an ein Polymer der Einzelpolymerschicht (12) gebunden ist und so konfiguriert ist, dass es ein lumineszierendes oder fluoreszierendes Reagenzsignal erzeugt, das auf das Vorhandensein des Zielobjekts innerhalb der Einzelpolymerschicht (12) hinweist, wenn die Einzelpolymerschicht (12) mit Anregungslicht (30) aus einer Anregungsquelle (28) bestrahlt wird, ii) ein optisches Isoliermittel (40) und iii) eine optische Referenz (38), die kovalent an ein Polymer der Einzelpolymerschicht (12) gebunden ist und so konfiguriert ist, dass sie ein optisches Referenzsignal erzeugt, das im Allgemeinen unabhängig vom Vorhandensein des Zielobjekts innerhalb der Einzelpolymerschicht (12) ist, wenn die Einzelpolymerschicht (12) mit dem Anregungslicht (30) aus der Anregungsquelle (28) bestrahlt wird, und wobei die Einzelpolymerschicht (12) iv) mindestens semipermeabel für das Zielobjekt ist, v) eine erste Oberfläche (26) aufweist, die so konfiguriert ist, dass sie mit dem in der Detektionskammer (14) vorhandenen Probenfluid (20) in Kontakt tritt, vi) eine zweite Oberfläche (22) aufweist, die an einem Träger befestigt ist, der bei Wellenlängen, die zum Anregen und Detektieren von Lumineszenz oder Fluoreszenz von dem lumineszierenden oder fluoreszierenden Reagenz (36) und/oder der darin vorhandenen optischen Referenz (38) geeignet sind, optisch transparent ist, und vii) einen Durchlässigkeitsgrad von etwa 20 % oder weniger bei einer Spitzenanregungswellenlänge des lumineszierenden oder fluoreszierenden Reagenz (36) entlang einer optischen Achse aufweist, die senkrecht zur zweiten Oberfläche (22) der Einzelpolymerschicht (12) steht.

2. Optischer Sensor nach Anspruch 1, wobei die Einzelpolymerschicht im trockenen Zustand eine Dicke von etwa 75 µm oder weniger entlang der optischen Achse senkrecht zur zweiten Oberfläche (22) der Einzelpolymerschicht (12) aufweist.

3. Optischer Sensor (10) nach Anspruch 1 oder 2, wobei die Einzelpolymerschicht (12) im trockenen Zustand eine Dicke von mindestens etwa 20 µm entlang der optischen Achse senkrecht zur zweiten Oberfläche (22) der Einzelpolymerschicht (12) aufweist.

4. Optischer Sensor (10) nach einem der vorstehenden Ansprüche, wobei die Detektionskammer (14) eine erste Wand (18) umfasst, die bei der maximalen Anregungswellenlänge des lumineszierenden oder fluoreszierenden Reagenz (36) transparent ist, die zweite Oberfläche (22) der Einzelpolymerschicht (12) an einer Innenoberfläche der ersten Wand (18) der Detektionskammer (14) angeordnet ist und die optische Achse senkrecht zur zweiten Oberfläche (22) durch die erste Wand (18) in die Einzelpolymerschicht (12) verläuft.

5. Optischer Sensor (10) nach einem der vorstehenden Ansprüche, wobei die Einzelpolymerschicht (12) Ruß, Kohlenstoffnanostrukturen und/oder einen nichtfluoreszierenden Farbstoff umfasst.

6. Optischer Sensor (10) nach einem der vorstehenden Ansprüche, wobei das Probenfluid (20) Blut, Plasma oder Serum umfasst.

7. Optischer Sensor (10) nach einem der vorstehenden Ansprüche, wobei das Zielobjekt aus einer Gruppe bestehend aus Ca⁺⁺, K⁺, Na⁺oder H*⁺*, Kreatinin, Laktat und Glukose ausgewählt ist.

8. Verfahren zum Detektieren eines in einen Probenfluid vorhandenen Zielobjekts (20), wobei das Verfahren umfasst:
Kontaktieren einer ersten Oberfläche (26) einer Einzelpolymerschicht (12) mit dem Probenfluid (20), wobei die Einzelpolymerschicht (12) für das Zielobjekt mindestens semipermeabel ist und i) ein lumineszierendes oder fluoreszierendes Reagenz (36), das kovalent an ein Polymer der Einzelpolymerschicht (12) gebunden ist und so konfiguriert ist, dass es ein lumineszierendes oder fluoreszierendes Reagenzsignal erzeugt, das auf das Vorhandensein des Zielobjekts innerhalb der Einzelpolymerschicht (12) hinweist, wenn die Einzelpolymerschicht (12) mit Anregungslicht (30) aus einer Anregungsquelle (28) bestrahlt wird, ii) ein optisches Isoliermittel (40) und iii) eine optische Referenz (38) umfasst, die kovalent an ein Polymer der Einzelpolymerschicht (12) gebunden ist und so konfiguriert ist, dass sie ein optisches Referenzsignal erzeugt, das im Allgemeinen unabhängig vom Vorhandensein des Zielobjekts innerhalb des Probenfluids (20) ist, wenn die Einzelpolymerschicht (12) mit dem Anregungslicht (30) aus der Anregungsquelle (28) bestrahlt wird;
Bestrahlen der Einzelpolymerschicht (12) mit dem Anregungslicht (30) entlang einer optischen Achse, die durch eine zweite Oberfläche (22) der Einzelpolymerschicht (12), die nicht mit dem Probenfluid in Kontakt steht, in die Einzelpolymerschicht (12) verläuft, wobei die optische Achse senkrecht zur zweiten Oberfläche (22) der Einzelpolymerschicht (12) steht, wobei die zweite Oberfläche (22) auf einem Träger befestigt ist, der bei Wellenlängen, die zum Anregen und Detektieren von Lumineszenz oder Fluoreszenz eines lumineszierenden oder fluoreszierenden Reagenz (36) und/oder eine darin vorhandene optische Referenz (38) geeignet sind, optisch transparent ist, und
die Einzelpolymerschicht (12) bei einer maximalen Anregungswellenlänge des lumineszierenden oder fluoreszierenden Reagenz (36) entlang der optischen Achse etwa 20% oder weniger durchlässt.

9. Verfahren nach Anspruch 8, wobei im Wesentlichen die gesamte detektierte Fluoreszenz von dem lumineszierenden oder fluoreszierenden Reagenz (36) oder der optischen Referenz (38) in der Einzelpolymerschicht (12) herrührt.

10. Verfahren nach Anspruch 8 oder 9, wobei das Zielobjekt aus einer Gruppe bestehend aus Ca⁺⁺, K⁺, Na⁺oder H⁺, Kreatinin, Laktat und Glukose ausgewählt ist.

11. Verfahren nach einem der Ansprüche 8-10, wobei die Einzelpolymerschicht (12) Ruß, Kohlenstoffnanostrukturen und/oder ein nichtfluoreszierendes Material umfasst.

12. Verfahren nach einem der Ansprüche 8-11, wobei die Einzelpolymerschicht (12) im trockenen Zustand eine Dicke von etwa 75 µm oder weniger aufweist.

13. Verfahren nach einem der Ansprüche 8-12, wobei die Einzelpolymerschicht (12) im trockenen Zustand eine Dicke von mindestens etwa 20 µm aufweist.

## Revendications

1. Capteur optique (10) pour détecter une cible présente dans un fluide échantillon (20), comprenant :
une chambre de détection (14) configurée pour recevoir le fluide échantillon (20) ;
une couche de polymère unique (12) disposée dans la chambre de détection (14), la couche de polymère unique (12) comprenant :
i) un réactif luminescent ou fluorescent (36) qui est lié de manière covalente à un polymère de la couche de polymère unique (12) et est configuré pour générer un signal de réactif luminescent ou fluorescent indiquant la présence de la cible au sein de la couche de polymère unique (12) lorsque la couche de polymère unique (12) est irradiée par la lumière d'excitation (30) provenant d'une source d'excitation (28), ii) un agent d'isolation optique (40), et iii) une référence optique (38) qui est liée de manière covalente à un polymère de la couche de polymère unique (12) et est configurée pour générer un signal de référence optique généralement indépendant de la présence de la cible au sein de la couche de polymère unique (12) lorsque la couche de polymère unique (12) est irradiée par la lumière d'excitation (30) provenant de la source d'excitation (28), et dans lequel la couche de polymère unique (12) iv) est au moins semi-perméable à la cible, v) présente une première surface (26) configurée pour entrer en contact avec le fluide échantillon (20) présent dans la chambre de détection (14), vi) présente une seconde surface (22) fixée à un support qui est optiquement transparent aux longueurs d'onde utiles pour exciter et détecter la luminescence ou la fluorescence du réactif luminescent ou fluorescent (36) et/ou la référence optique (38) présente dans celle-ci, et vii) présente une transmittance d'environ 20 % ou moins à une longueur d'onde d'excitation maximale du réactif luminescent ou fluorescent (36) le long d'un axe optique qui est normal à la seconde surface (22) de la couche de polymère unique (12).

2. Capteur optique selon la revendication 1, dans lequel la couche de polymère unique présente une épaisseur à l'état sec d'environ 75 µm ou moins le long de l'axe optique normal à la seconde surface (22) de la couche de polymère unique (12).

3. Capteur optique (10) selon la revendication 1 ou 2, dans lequel la couche de polymère unique (12) présente une épaisseur à l'état sec d'au moins environ 20 µm le long de l'axe optique normal à la seconde surface (22) de la couche de polymère unique (12).

4. Capteur optique (10) selon l'une quelconque des revendications précédentes, dans lequel la chambre de détection (14) comprend une première paroi (18) qui est transparente à la longueur d'onde d'excitation maximale du réactif luminescent ou fluorescent (36), la seconde surface (22) de la couche de polymère unique (12) est disposée contre une surface intérieure de la première paroi (18) de la chambre de détection (14), et l'axe optique normal à la seconde surface (22) passe à travers la première paroi (18) dans la couche de polymère unique (12).

5. Capteur optique (10) selon l'une quelconque des revendications précédentes, dans lequel la couche polymère unique (12) comprend du noir de carbone, des nanostructures de carbone, et/ou un colorant non fluorescent.

6. Capteur optique (10) selon l'une quelconque des revendications précédentes, dans lequel le fluide échantillon (20) comprend du sang, du plasma ou du sérum.

7. Capteur optique (10) selon l'une quelconque des revendications précédentes, dans lequel la cible est sélectionnée parmi un groupe consistant en Ca⁺⁺, K⁺, Na⁺, ou H⁺, la créatinine, le lactate et le glucose.

8. Procédé de détection d'une cible présente dans un fluide échantillon (20), le procédé comprenant :
la mise en contact d'une première surface (26) d'une couche de polymère unique (12) avec le fluide échantillon (20), la couche de polymère unique (12) étant au moins semi-perméable à la cible et comprenant i) un réactif luminescent ou fluorescent (36) lié de manière covalente à un polymère de la couche de polymère unique (12) et configuré pour générer un signal de réactif luminescent ou fluorescent indiquant la présence de la cible au sein de la couche de polymère unique (12) lorsque la couche de polymère unique (12) est irradiée par la lumière d'excitation (30) provenant d'une source d'excitation (28), ii) un agent d'isolation optique (40), et iii) une référence optique (38) liée de manière covalente à un polymère de la couche de polymère unique (12) et configurée pour générer un signal de référence optique généralement indépendant de la présence de la cible au sein du fluide échantillon (20) lorsque la couche de polymère unique (12) est irradiée par la lumière d'excitation (30) provenant de la source d'excitation (28),
l'irradiation de la couche de polymère unique (12) avec la lumière d'excitation (30) le long d'un axe optique qui pénètre dans la couche de polymère unique (12) à travers une seconde surface (22) de la couche de polymère unique (12) non en contact avec le fluide échantillon, l'axe optique étant normal à la seconde surface (22) de la couche de polymère unique (12), dans lequel la seconde surface (22) est fixée à un support qui est optiquement transparent aux longueurs d'onde utiles pour exciter et détecter la luminescence ou la fluorescence d'un réactif luminescent ou fluorescent (36) et/ou d'une référence optique (38) présente dans celle-ci, et
la couche de polymère unique (12) transmettant environ 20 % ou moins à une longueur d'onde d'excitation maximale du réactif luminescent ou fluorescent (36) le long de l'axe optique.

9. Procédé selon la revendication 8, dans lequel la quasi-totalité de la fluorescence détectée provient du réactif luminescent ou fluorescent (36) ou de la référence optique (38) dans la couche de polymère unique (12).

10. Procédé selon la revendication 8 ou 9, dans lequel la cible est sélectionnée parmi un groupe consistant en Ca⁺⁺, K⁺, Na⁺, ou H⁺, la créatinine, le lactate et le glucose.

11. Procédé selon l'une quelconque des revendications 8-10, dans lequel la couche polymère unique (12) comprend du noir de carbone, des nanostructures de carbone, et/ou un colorant non fluorescent.

12. Procédé selon l'une quelconque des revendications 8-11, dans lequel la couche de polymère unique (12) présente une épaisseur, à l'état sec, d'environ 75 µm ou moins.

13. Procédé selon l'une quelconque des revendications 8-12, dans lequel la couche de polymère unique (12) présente une épaisseur, à l'état sec, d'au moins environ 20 µm.
